# EUROPEAN PATENT APPLICATION

(11) **EP 0 811 378 A1**
(43) Date of publication of application: **10.12.1997**
(21) Application number: 96902481.9
(22) Date of filing: 19.02.1996
(51) Int. Cl.: A61K 31/435, C07D 455/02

(54) **PREVENTIVE/REMEDY FOR INTERSTITIAL PNEUMONIA, INFLAMMATORY INTESTINAL DISEASES OR VASCULAR THICKENING CONTAINING QUINOLIZINONE COMPOUNDS, SALT THEREOF OR HYDRATES THEREFOR**

(30) Priority: 23.02.1995 JP 35662/95; 02.11.1995 JP 285925/95; 27.12.1995 JP 341511/95
(71) Applicant: FUJISAWA PHARMACEUTICAL CO., LTD., Osaka-shi Osaka 541 (JP)
(72) Inventor: HIROI, Jun, Osaka 589 (JP); HATANO, Kazuo, Kyoto 617 (JP); NAKAI, Toru, Hyogo 663 (JP); MASUNAGA, Taro, Hyogo 663 (JP); KURATANI, Kazuyoshi, Kyoto-shi-Kyoto 612 (JP); TOMOI, Masaaki, Osaka 578 (JP)
(74) Representative: Türk, Gille, Hrabal, Leifert
(86) International application number: JP9600371
(87) International publication number: WO9625932

(57) **Abstract**

A preventive/remedy for interstitial pneumonia, inflammatory intestinal diseases or vascular thickening which contains as the active ingredient quinolizinone compounds represented by general formula (I), salts thereof or hydrates thereof wherein R¹ represents amidated carboxy, etc.; R⁴ represents hydrogen, etc.; R² represents hydrogen, etc.; and R³ represents aryloxy, etc., provided that R² and R³ may be located each at an arbitrary position of the quinolidine ring and bonded to each other to thereby form -CH₂CH₂CH₂-, etc.

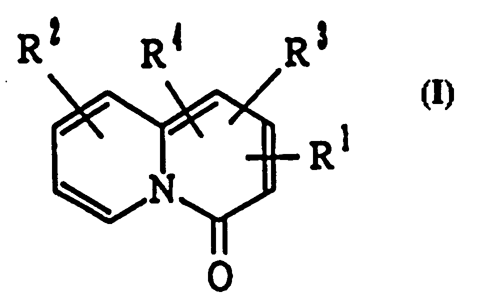

## Description

### TECHNICAL FIELD

This invention relates to a prophylactic and therapeutic agent for interstitial pneumonia, inflammatory bowel diseases, or vascular hypertrophy. More particularly, this invention relates to a prophylactic and therapeutic agent to be indicated in interstitial pneumonia, inflammatory bowel diseases, or vascular hypertrophy, which agent comprises a quinolizinone compound of the following formula, a salt thereof or a hydrate thereof, as an active ingredient, which finds application in the field of medicine. 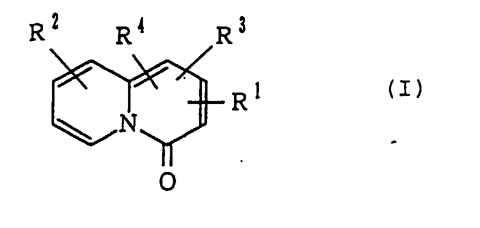 wherein
R¹ represents carboxy, amidated carboxy, cyano, thiocarbamoyl, or tetrazolyl;
R⁴ represents hydrogen or aryl;
R² represents hydrogen, hydroxy, lower alkyl, or lower alkoxy;
R³ represents hydrogen, hydroxy, lower alkyl, lower alkoxy, lower alkenyloxy, aryl that may have suitable substituent(s), arylthio, aroyl, ar(lower)alkyl, arenesulfonyl, arylamino that may have suitable substituent(s) or aryloxy;
R² and R³ may occur in any substitutable positions of the quinolizinone ring and may be bound to each other to form -CH₂CH₂CH₂-, -CH=CH-, or -CH=CH-CH=CH-).

### BACKGROUND TECHNOLOGY

The quinolizinone compound (I) for the practice of this invention is known per se, and it is disclosed in Japanese Kokai Tokkyo Koho S60-222482 that the compound is of value as an antiallergic agent as well as an antiulcer agent and in Japanese Kokai Tokkyo Koho H6-256187 that it is of use as an antitussive-expectorant.

It is also known that this quinolizinone compound (I) is of value as an analgesic agent, an anticonjunctivitis agent, or an anti-rheumatoid arthritic agent (International publication No. WO 94/07491).

Meanwhile, "interstitial pneumonia" is a class of diseases running the whole gamut of lung diseases with principal lesions in the alveolar interstitium (the connective tissue matrix bordered by the alveolar epithelial basement membrane and containing connective tissue fiber components such as collagen, elastin, proteoglycans, etc., capillaries, fibroblast cells, etc., which is important as a place of gas exchange) of the lung.

Interstitial pneumonia is characterized by an increased population of inflammatory cells (alveolar macrophages, lymphocytes, neutrophils, eosinophils, etc.) in the alveoli, a sign of the so-called alveolitis. In alveolitis, tissue injuries occur owing to the active oxygen, protease, and cytotoxic protein produced and released by inflammatory cells, thus motivating a repair function to work and, though the degree of repair varies, triggering a fibrotic process. In interstitial pneumonia, fibrosis plays havoc with said place of gas exchange, causing respiratory insufficiency.

Interstitial pneumonia is a disease of poor prognosis and no effective prophylactic or therapeutic modality has been available as yet.

In Crohn's disease and ulcerative colitis which are refractive chronic inflammatory bowel diseases, antiinflammatory sulfa drugs such as sulfasalazine, adrenocorticoids including prednisolone, and immunosuppressants such as azathioprine have been clinically employed.

However, these drugs are not necessarily satisfactory in adverse reaction potential and efficacy and, therefore, a true need has been felt for the development of a prophylactic and therapeutic agent with sufficient efficacy and low adverse reaction potential that can be judiciously indicated in inflammatory bowel diseases.

It is further known that a surgical operation such as angioplasty, arterial bypass surgery, or organ transplantation often entails vascular hypertrophy and obliteration due to the migration and proliferation of vascular smooth muscle cells. Though extensive research is going on for the development of prophylactic and therapeutic drugs for these disorders, no effective pharmacotherapies are known as of the present time.

The inventors of this invention did much research and unveiled the potential of said quinolizinone compound (I), a salt thereof or a hydrate thereof to prevent and cure vascular hypertrophy. Then, they anticipated the usefulness of said quinolizinone compound (I), salt and hydrate in mast cell-associated diseases such as hepatitis, liver sclerosis, ulcerative colitis, renal failure, pulmonary fibrosis, osteoporosis, etc., and proceeding with further research, discovered that said substances are of great value as a prophylactic and therapeutic agent not only for interstitial pneumonia inclusive of pulmonary fibrosis but for inflammatory bowel diseases inclusive of ulcerative colitis.

It is, therefore, the object of this invention to provide a prophylactic and therapeutic agent for interstitial pneumonia, inflammatory bowel diseases or vascular hypertrophy, which comprises said quinolizinone compound (I), a salt thereof or a hydrate thereof as an active ingredient.

### DISCLOSURE OF THE INVENTION

The detailed definitions of R¹, R², R³ and R⁴ of quinolizinone compound (I) and the preferred typical examples are shown below.

The term "lower" means 1-6 carbon atoms unless otherwise indicated.

The preferred "lower alkyl" and the "lower alkyl" moiety in "ar(lower)alkyl" include straight-chain or branched C₁₋₆ alkyl groups such as methyl, ethyl, propyl, isopropyl, butyl, isobutyl, tert-butyl, pentyl, and hexyl, among others.

The preferred "lower alkoxy" includes, methoxy, ethoxy, propoxy, isopropoxy, butoxy, tert-butoxy, pentyloxy and hexyloxy, among others, preferably one having 1-4 carbon atoms.

The preferred "lower alkenyloxy" includes vinyloxy, allyloxy, 1-propenyloxy, 1- or 2-butenyloxy, and 2-pentenyloxy, among others, preferably one having 2-4 carbon atoms.

The preferred "amidated carboxy" is an amide (-CONH₂) optionally having, on the nitrogen atom, suitable substituents such as heterocyclic groups optionally having suitable substituents and aryl groups optionally having suitable substituents as will be described hereinafter.

The "heterocyclic group" mentioned above includes heterocyclic groups each containing one or more hetero-atoms selected from among oxygen, sulfur, nitrogen, etc. in addition to carbon as ring members, whether saturated or unsaturated and whether monocyclic or polycyclic.

The preferred "heterocyclic group" includes but is not limited to the following:

Unsaturated 3- through 8-membered, preferably 5- or 6-membered, heteromonocyclic groups each containing 1-4 nitrogen atoms as ring members, such as pyrrolyl, pyrrolinyl, imidazolyl, pyrazolyl, pyridyl inclusive of its N-oxide, dihydropyridyl, pyrimidyl, pyrazinyl, pyridazinyl, triazolyl (e.g. 4H-1,2,4-triazolyl, 1H-1,2,3-triazolyl, 2H-1,2,3-triazolyl, etc.), tetrazolyl (e.g. 1H-tetrazolyl, 2H-tetrazolyl, etc.), triazinyl (e.g 1,2,3-triazinyl, 1,2,4-triazinyl, 1,3,5-triazinyl, etc.), etc.;

Saturated 3- through 8-membered, preferably 5- or 6-membered, heteromonocyclic groups each containing 1-4 nitrogen atoms as ring members, such as pyrrolidinyl, imidazolidinyl, piperidino, piperazinyl, etc.;

Unsaturated fused heterocyclic groups each containing 1-4 nitrogen atoms as ring members, such as indolyl, isoindolyl, indolidinyl, benzoimidazolyl, quinolyl, isoquinolyl, indazolyl, benzotriazolyl, tetrazolopyridyl, etc.;

Unsaturated 3- through 8-membered, preferably 5- or 6-membered, heteromonocyclic groups each containing 1-2 oxygen atoms and 1-3 nitrogen atoms as ring members, such as oxazolyl, isoxazolyl, oxadiazolyl (e.g 1,2,4-oxadiazolyl, 1,3,4-oxadiazolyl, 1,2,5-oxadiazolyl, etc.), etc.;

Saturated 3- through 8-membered, preferably 5- or 6-membered, heteromonocyclic groups each containing 1-2 oxygen atoms and 1-3 nitrogen atoms as ring members, such as morpholinyl, sydnonyl, etc,;

Unsaturated fused heterocyclic groups each containing 1-2 oxygen atoms and 1-3 nitrogen atoms as ring members, such as benzoxazolyl, benzoxadiazolyl, etc.;

Saturated 3- through 8-membered, preferably 5- or 6-membered, heteromonocyclic groups each containing 1-2 sulfur atoms and 1-3 nitrogen atoms as ring members, such as thiazolidinyl, etc.;

Unsaturated 3- through 8-membered, preferably 5- or 6-membered, heteromonocyclic groups each containing 1-2 sulfur atoms as ring members, such as thienyl, dihydrodithiinyl, dihydrodithionyl, etc.;

Unsaturated fused heterocyclic groups each containing 1-2 sulfur atoms and 1-3 nitrogen atoms as ring members, such as benzothiazolyl, benzothiadiazolyl, etc.;

Unsaturated 3- through 8-membered, preferably 5- or 6-membered, heteromonocyclic groups each containing one oxygen atom as a ring member, such as furyl etc.,

Unsaturated 3- through 8-membered, preferably 5- or 6-membered, heteromonocyclic groups each containing one oxygen atom and 1-2 sulfur atoms as ring members, such as dihydrooxathiinyl etc.,

Unsaturated fused heterocyclic groups each containing 1-2 sulfur atoms as ring members, such as benzothienyl, benzodithiinyl, etc.;

Unsaturated fused heterocyclic groups each containing one oxygen atom and 1-2 sulfur atoms as ring members, such as benzoxathiinyl etc.

Each of the above-mentioned heterocyclic groups may have one or more substituents selected from among lower alkyl (e.g. methyl, ethyl, propyl, isopropyl, butyl, isobutyl, t-butyl, pentyl, neopentyl, hexyl, etc.), halogen (e.g. chlorine, bromine, fluorine, iodine), etc.

The preferred "aryl" includes phenyl, tolyl, xylyl, cumenyl, naphthyl, biphenyl, etc. and each of them may have one or more suitable substituents, such as amino, nitro, halogen (e.g. chlorine, bromine, fluorine, iodine), lower alkoxy mentioned above, carboxy, hydroxy, and lower alkoxycarbonyl (e.g methoxycarbonyl, ethoxycarbonyl, etc.), among others.

The preferred "aryl" for said "arylthio", "ar(lower)alkyl" or "arylamino" includes the species of aryl mentioned above.

The preferred "aroyl" includes benzoyl, toluoyl, naphthoyl, etc.

The preferred "arenesulfonyl" includes benzenesulfonyl, p-toluenesulfonyl, etc.

The "arylamino" mentioned above may have suitable substituents such as lower alkyl (e.g methyl, ethyl, etc.) on the nitrogen atom.

The preferred "aryloxy" includes phenoxy, tolyloxy, etc., although phenoxy is particularly preferred.

The preferred "amidated carboxy" includes carbamoyl, pyridylamido, pyrimidinylamido optionally having lower alkyl, pyrazinylamido, phenylamido optionally having hydroxy, thiazolylamido, triazinylamido, triazolylamido, pyridazinylamido optionally having halogen, tetrazolylamido, etc. Among them, tetrazolylamido is particularly preferred.

The preferred salt of quinolizinone compound (I) includes medicinally acceptable salts, particularly nontoxic salts with ordinary bases and nontoxic acid addition salts. Thus, salts with inorganic bases (e.g alkali metal salts such as sodium salt, potassium salt, etc.) or alkaline earth metal salts (e.g. calcium salt, magnesium salt, etc.); ammonium salts; salts with organic bases such as triethylamine salt, pyridine salt, picoline salt, ethanolamine salt, triethanolamine salt, dicyclohexylamine salt, N,N'-dibenzylethylenediamine salt, etc.; inorganic acid addition salts such as hydrochloride, hydrobromide, sulfate, phosphate, etc.; organic carboxylic or sulfonic acid addition salts such as formate, acetate, trifluoroacetate, maleate, tartrate, methanesulfonate, benzenesulfonate, p-toluenesulfonate, etc.; and salts with basic or acidic amino acids such as arginine, aspartic acid, glutamic acid, etc. can be mentioned.

Among those salts, the alkali metal salts are preferred and the sodium salt is particularly preferred.

The hydrate of quinolizinone compound (I) or a salt thereof includes the mono-, tetra-, and other hydrates, although the monohydrate is preferred.

As the active ingredient of the prophylactic and therapeutic agent of the invention for interstitial pneumonia, inflammatory bowel diseases or vascular hypertrophy, the species of quinolizinone compound (I) wherein R¹ is tetrazolylcarbamoyl, R² is hydrogen, R³ is phenoxy, and R⁴ is hydrogen, or a salt thereof, or a hydrate thereof, is preferred and N-[5-(1H-tetrazolyl)]-1-phenoxy-4H-quinolizin-4-one-3-carboxamide sodium salt (II) and a hydrate, particularly the monohydrate, thereof, is the most useful.

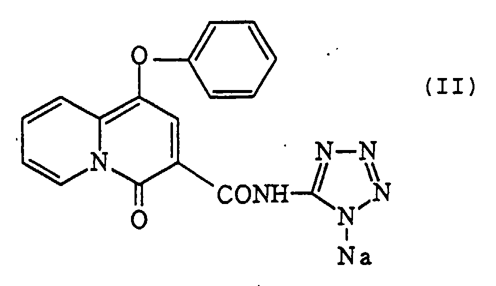

The above quinolizinone compound (I) and salt can be produced by several processes known per se. Such processes are described in detail in Japanese Kokai Tokkyo Koho S60-222482. The hydrate of the compound (II) and a process for its production are described in detail in Japanese Kokai Tokkyo Koho H1-104073.

The prophylactic and therapeutic agent of this invention for interstitial pneumonia, inflammatory intestinal disease or vascular hypertrophy can be put to use in a variety of pharmaceutical dosage forms including solid, semisolid and liquid dosage forms as prepared by formulating the active ingredient of the invention with pharmaceutically acceptable carriers compatible with oral or parenteral administration such as inorganic and/or organic solid or liquid excipients and the like. The active ingredient can be formulated with nontoxic medicinally acceptable carriers which are usually employed and compatible with capsules, tablets, dragees, solutions, suspensions, emulsions, aerosols or other drug delivery systems. The carrier that can be used includes not only water, glucose, lactose, gum arabic, gelatin, mannitol, starch paste, polyvinylpyrrolidone, magnesium stearate, talc, corn starch, keratin, colloidal silica, potato starch, urea, chlorofluorocarbon 11, etc. but also other carriers suitable for the manufacture of solid, semisolid, and liquid drug products. In addition to such carriers, any other suitable auxiliaries, stabilizers, thickeners, buffers, dispersants, colorants, and flavoring agents can be incorporated. In order that the active ingredient may retain its activity within the dosage form, this pharmaceutical composition may be supplemented with preservatives, bacteriostatic agents or the like. The amount of the active quinolizinone compound (I) in the pharmaceutical composition should be just large enough to insure the desired therapeutic effect depending on the course and condition of the disease.

For application of this pharmaceutical composition to man, the preferred routes of administration are oral, by inhalation, intravenous, intramuscular, etc. Though the dosage or therapeutically effective amount of quinolizinone compound (I) depends on the type and severity of the disease to be treated, the patient's age and other factors, etc., the therapeutic daily dose for man and animals is usually about 0.1-10 mg/kg. Generally, this dose is administered 1-3 times a day.

Below presented are some pharmacological test data demonstrating the usefulness of the quinolizinone compound, salt or hydrate as a prophylactic and therapeutic agent for interstial pneumonia, inflammatory bowel diseases or vascular hypertrophy.

### [1] Test compound

(1) N-[5-(1H-tetrazolyl)]-1-phenoxy-4H-quinolizin-4-one-3-carboxamide sodium salt (hereinafter referred to as "test compound 1")
(2) Tranilast (a known antiallergic drug)
(3) N-[5-(1H-tetrazolyl)]-1-phenoxy-4H-quinolizin-4-one-3-carboxamide sodium salt monohydrate (hereinafter referred to as "test compound 2")

### [2] Experiment

### (A) Efficacy in a mouse model of bleomycin-induced pulmonary fibrosis

### (1) Method

ICR mice (12 weeks old, male gender) were intravenously dosed with bleomycin (10 mg/kg) daily for 10 days. Test compound 1 was suspended in 0.5% methylcellulose solution and administered orally twice a day beginning the first day of bleomycin treatment till the day immediately preceding sacrifice. A control group received 0.5% methylcellulose solution in the like manner.

Twenty-eight (28) days reckoning from administration of the initial dose of bleomycin, the mice were sacrificed, the lungs were isolated, and the lung hydroxyproline content was determined in accordance with Woessner's method.

Histopathological specimens were also prepared at the same time and the degree of fibrotic change was evaluated after Azan-Mallory staining.

### (2) Results

The results are presented in Table 1.

**Table 1**

| | Dosage (mg/kg) | n | Hydroxyproline content (µg/lung) | Degree of inhibition (%) |
|---|---|---|---|---|
| Control group | | 10 | 440.5±13.8 | |
| Test compound 1 group | 1 | 10 | 374.4*±8.3 | 88.6 |
| Intact group | | 10 | 365.9**±13.7 | |
| Mean ± SE | | | | |

| | | | | |
|---|---|---|---|---|
| *, **: significantly different from control group at 5% and 1% levels (ANOVA followed by Tukey-Kramar multiple comparison) | | | | |

The histopathological examination revealed a definite inhibition of pulmonary fibrosis in Test compound 1 group in parallel with the inhibition of hydroxyproline content.

### (B) Efficacy in a mouse model of silica-induced pulmonary fibrosis

### (1) Method

Using C57BL/6 mice (8 weeks old, male gender), the neck was incised under pentobarbital anesthesia and silica (2 mg/0.1 ml/animal) was administered intra-tracheally. A sham operated intact group received physiological saline in the same manner. The drug was orally administered daily beginning the day of silica treatment till the day immediately preceding sacrifice. Thus, test compound 1 and tranilast were respectively administered once after silica treatment and twice daily on the next and subsequent days, in morning and evening. In the sham operated and control groups, 0.5% methylcellulose was similarly administered.

Sixteen (16) days after sensitization, the mice were bled to death under pentobarbital anesthesia and the lungs were isolated. The hydroxyproline content of the isolated lungs was assayed and used as an indicator of pulmonary fibrosis. The difference in mean value between the control group and the treatment group or the sham operated group was tested by one-way analysis of variance and Dunnett multiple-comparison test.

### (2) Results

The results are presented in Table 2.

**Table 2**

| | Dosage (mg/kg) | n | Hydroxyproline content (µg/lung) | Degree of inhibition (%) |
|---|---|---|---|---|
| Control group | | 10 | 243.19±16.38 | |
| Test compound 1 group | 1 | 10 | **144.41±9.69 | 88.7 |
| Tranilast group | 100 | 9 | 224.66±18.44 | 13.6 |
| Sham-operated group | | 10 | **131.81±7.47 | |
| Mean ± SE | | | | |

| | | | | |
|---|---|---|---|---|
| **: significantly different from control group at the 1% level (one-way analysis of variance and Dunnett multiple-comparison test) | | | | |

### (C) Efficacy in a rat model of acetic acid-induced colitis

### (1) Method

Male SD rats (7-8 weeks old) were deprived of food for 24 hours and a suspension of test compound 2 in 0.5% methylcellulose solution was administered orally. Thirty (30) minutes later, 1 ml/rat of 4% acetic acid in saline was infused from the anus under ether anesthesia. An intact group received a saline infusion. The infusion was carried out over 20 seconds. Then, the animal was held in inverted upright position for 30 seconds, after which 2 ml of phosphate buffer solution was infused for washout. Twenty-four (24) hours later, the animal was sacrificed by carbon dioxide inhalation, the large intestine was isolated, and the local lesion was scored according to the following criteria. Lesion score
- 0.: No lesion
- 1.: Hyperemia without ulceration
- 2.: Inflammation (hyperemia and thickening of the bowel wall) or ulceration without definite inflammation
- 3.: Ulceration with inflammation at 1 site
- 4.: Ulceration with inflammation at 2 or more sites
- 5.: A large ulcer sized 1 cm or more
- 6-10.: An ulcer sized 2 cm or more; 1 point added for each increment of 1 cm

### (2) Results

The results are presented in Table 3.

**Table 3**

| | Dosage (mg/kg) | n | Lesion score | Degree of inhibition (%) |
|---|---|---|---|---|
| Control group | | 10 | 4.70±0.15 | |
| Test compound 2 group | 1 | 10 | 2.90*±0.41 | 38.3 |
| | 10 | 10 | 1.50***±0.31 | 68.1 |
| Mean ± S.E. | | | | |

| | | | | |
|---|---|---|---|---|
| *, ***: significantly different from control group at the 5% and 0.1% levels (Kruskal-Wallis nonparametric analysis of variance and Dunn multiple-comparison test) | | | | |

Incidentally, the oral acute toxicity LD₅₀ value of test compound 2 in rats was 4.5 g/kg/day for male gender and 5 g/kg/day or more for female gender.

### INDUSTRIAL APPLICABILITY

As apparent from the experimental data given under (A) and (B), the quinolizinone compound (I), a salt thereof, and a hydrate thereof are of value as a prophylactic and therapeutic agent for various types of interstitial preumonia [e.g. occupational interstitial lung diseases (such as pneumoconiosis, silicosis, asbestosis, etc.), hypersensitivity pneumonitis (summer hypersensitivity pneumonia, farmer's lung, etc.), drug-induced pneumonia (e.g. pneumonia as a side effect of bleomycin), radiopneumonitis, infective lung diseases (miliary tuberculosis, mycoplasmal pneumonia, etc.), heart disease- or malignant tumor-associated pneumonia, pulmonary fibrosis, collagen disease-associated interstitial pneumonia, sarcoidosis, eosinophilic granuloma, pulmonary alveolar proteinosis, pulmonary hemosiderosis, Goodpasture's syndrome, etc.].

It is also apparent from the experimental data presented under (C), the quinolizinone compound (I), a salt thereof and their hydrades are also of value as a prophylactic and therapeutic agent for a variety of inflammatory bowel diseases (e.g. Crohn's disease, ulcerative colitis, etc.).

The quinolizinone compound (I), a salt thereof and their hydrades are further expected to be of value as a prophylactic and therapeutic agent for the vascular hyperplasia, obliteration or restenosis primarily associated with the migration and proliferation of vascular smooth muscle cells following various types of angioplasty (e.g. percutaneous coronary angioplasty), arterial bypass surgery, tissue (organ) transplantation, etc. and still further as a prophylactic and therapeutic agent for atherosclerosis.

### Example 1

Tablets were manufactured according to the following recipe per tablet.

| | |
|---|---|
| N-[5-(1H-tetrazolyl)]-1-phenoxy-4H-quinolizin-4-one-3-carboxamide sodium salt (active ingredient) | 25 mg |
| Lactose | 150 mg |
| Corn starch | 60 mg |
| Polyvinylpyrrolidone | 10 mg |
| Magnesium stearate | 5 mg |

The active ingredient, lactose and corn starch were thoroughly blended, wetted with a 20% solution of polyvinylpyrrolidone in ether, dried at 45°C, and passed through a 20-mesh sieve. The granule thus obtained was mixed with magnesium stearate and the resulting composition was compressed into tablets.

### Example 2

Tablets were manufactured according to the following recipe per tablet.

| | |
|---|---|
| N-[5-(1H-tetrazolyl)]-1-phenoxy-4H-quinolizin-4-one-3-carboxamide sodium salt monohydrate (active ingredient) | 25 mg |
| Lactose | 150 mg |
| Corn starch | 60 mg |
| Polyvinylpyrrolidone | 10 mg |
| Magnesium stearate | 5 mg |

The active ingredient, lactose and corn starch were thoroughly blended, wetted with a 20% solution of polyvinylpyrrolidone in ether, dried at 45°C, and passed through a 20-mesh sieve. The granule thus obtained was mixed with magnesium stearate and the resulting composition was compressed into tablets.

## Claims

1. A prophylactic and therapeutic agent for interstitial pneumonia, inflammatory bowel diseases or vascular hypertrophy, comprising a quinolizinone compound of the following formula, a salt thereof or a hydrate thereof, as an active ingredient. 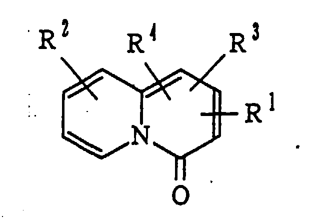 wherein
R¹ represents carboxy, amidated carboxy, cyano, thiocarbamoyl, or tetrazolyl;
R⁴ represents hydrogen or aryl;
R² represents hydrogen, hydroxy, lower alkyl, or lower alkoxy;
R³ represents hydrogen, hydroxy, lower alkyl, lower alkoxy, lower alkenyloxy, aryl that may have suitable substituent(s), arylthio, aroyl, ar(lower)alkyl, arenesulfonyl, arylamino that may have suitable substituent(s) or aryloxy;
R² and R³ may occur in any substitutable positions of the quinolizinone ring and may be bound to each other to form -CH₂CH₂CH₂-, -CH=CH-, or -CH=CH-CH=CH-).

2. A prophylactic and therapeutic agent for interstitial pneumonia, inflammatory bowel diseases or vascular hypertrophy as claimed in Claim 1, comprising a quinolizinone compound of the formula wherein R¹ is tetrazolylcarbamoyl, R² is hydrogen, R³ is phenoxy, and R⁴ is hydrogen, a salt thereof or a hydrate thereof, as an active ingredient.

3. A prophylactic and therapeutic agnet for interstitial pneumonia, inflammatory bowel diseases or vascular hypertrophy as claimed in Claim 2, comprising N-[5-(1H-tetrazolyl)]-1-phenoxy-4H-quinolizin-4-one-3-carboxamide sodium salt or a hydrate thereof as an active ingredient.

4. A use of the compound of claim 1 or 2 or 3 as a prophylactic and therapeutic agent for interstitial pneumonia, inflammatory bowel diseases or vascular hypertrophy.

5. A use of the compound of claim 1 or 2 or 3 for manufacturing a medicament for preventing and treating interstitial pneumonia, inflammatory bowel diseases or vascular hypertrophy.

6. A pharmaceutical composition for interstitial pneumonia, inflammatory bowel diseases or vascular hypertrophy, which comprises the compound of claim 1 or 2 or 3 as an active ingredient.

7. A method for preventing and treating interstitial pneumonia, inflammatory bowel diseases or vascular hypertrophy, which comprises administering the compound of claim 1 or 2 or 3 to human or animals.
